# EUROPEAN PATENT SPECIFICATION

(11) **EP 3 938 040 B1**
(45) Date of publication and mention of the grant of the patent: **21.05.2025**
(21) Application number: 20717988.8
(22) Date of filing: 11.03.2020
(51) Int. Cl.: A61K 31/122, A61K 45/06, A61K 31/422, A61K 31/437, A61K 31/5377, A61K 31/4545, A61P 7/02, A61K 31/4439

(54) **VITAMIN K2 IN COMBINATION WITH ANTICOAGULANTS FOR USE IN TREATING MUSCLE SORENESS**
VITAMIN K2 IN KOMBINATION MIT ANTIKOAGULANTIEN ZUR BEHANDLUNG VON MUSKELKATER
VITAMINE K2 EN COMBINAISON AVEC DES ANTICOAGULANTS POUR LE TRAITEMENT DES DOULEURS MUSCULAIRES

(30) Priority: 12.03.2019 US 201962817037 P
(43) Date of publication of application: 19.01.2022
(73) Proprietor: Lesaffre et Compagnie, 75001 Paris (FR)
(72) Inventor: VAN GORP, Rick, F-75001 Paris (FR)
(74) Representative: Acapo AS
(86) International application number: PCT/NO2020/050066
(87) International publication number: WO 2020/185092

(56) References cited:
- US-A1- 2010 130 618
- US-A1- 2016 310 445
- ALMUTAIRI ABDULAALI R ET AL: "Effectiveness and Safety of Non-vitamin K Antagonist Oral Anticoagulants for Atrial Fibrillation and Venous Thromboembolism: A Systematic Review and Meta-analyses", CLINICAL THERAPEUTICS, EXCERPTA MEDICA, PRINCETON, NJ, US, vol. 39, no. 7, 28 June 2017 (2017-06-28), pages 1456, XP085141272, ISSN: 0149-2918, DOI: 10.1016/J.CLINTHERA.2017.05.358
- AN S. DE VRIESE ET AL: "Multicenter Randomized Controlled Trial of Vitamin K Antagonist Replacement by Rivaroxaban with or without Vitamin K2 in Hemodialysis Patients with Atrial Fibrillation: the Valkyrie Study", JOURNAL OF THE AMERICAN SOCIETY OF NEPHROLOGY., vol. 31, no. 1, 23 January 2020 (2020-01-23), US, pages 186 - 196, XP055702140, ISSN: 1046-6673, DOI: 10.1681/ASN.2019060579
- MCFARLIN BK: "Oral Consumption of Vitamin K2 for 8 Weeks Associated With Increased Maximal Cardiac Output During Exercise", ABSTRACT ALTERN THER HEALTH MED, 1 July 2017 (2017-07-01), pages 26 - 32, XP055697901, Retrieved from the Internet <URL:https://pdfs.semanticscholar.org/b7aa/3d24a5ab6c3ea387f4c7dae79d99f72d9c81.pdf?_ga=2.203169681.1491297138.1590410471-1060242777.1590410471> [retrieved on 20200525]

## Description

### CROSS REFERENCE TO RELATED APPLICATIONS

This application claims priority to U.S. Provisional Patent Application No. 62/817,037, filed March 12, 2019.

### TECHNICAL FIELD

The present disclosure is directed to the use of a composition for use in treating or preventing delayed onset muscle soreness, comprising administering to a subject in need thereof a composition comprising vitamin K2 and at least one anticoagulant. The present disclosure is also directed to a combination for use in treating or preventing delayed onset muscle soreness, comprising administering to a subject in need thereof a combination comprising vitamin K2 and at least one anticoagulant.

### BACKGROUND OF THE DISCLOSURE

Atrial fibrillation (Afib) is a serious condition characterized by an irregular and/or rapid heart rate. Symptoms of Afib include a fluttering or "thumping" in the chest, although the condition is often asymptomatic. Oftentimes, blood delivery to the body, including the heart, declines and/or becomes unpredictable as a result of Afib, which may lead to oxidative stress, muscle fatigue, and potentially heart attack. Furthermore, subjects suffering from Afib may be at an increased risk of forming blood clots in the heart, which may travel to other areas of the body, including the brain.

Blood thinners are traditionally used for the treatment of Afib, although such treatments generally only address reducing existing blood clots. There is thus still a need in the art for treating other symptoms of Afib, such as the oxidative stress that results from reduced blood flow, as well as a treatment for other conditions that may present with similar effects as those observed with Afib, such as reduced blood flow and low ATP production. Furthermore, because Afib is often asymptomatic, the most effective treatment would both prevent both clots and reduce oxidative stress.

McFarlin, B.K. et al., "Oral Consumption of Vitamin K2 for 8 Weeks Associated With Increased Maximal Cardiac Output During Exercise", Altern Ther Health Med. 2017 Jul;23(4):26-32, relates to a study of vitamin K2 to determine if dietary supplementation could increase the function of muscle with high mitochondrial content, i.e., skeletal and cardiac muscle.

US20100130618 discloses the therapeutic uses of vitamin K, its analogues and derivatives and vitamin K activity synthesized molecules to improve blood perfusion and ameliorate hypoxia for the prevention and/or treatment of chronic venous insufficiencies and consequent diverse complications such as venous and lymphatic oedema, melanization, paper-money skin, desquamation, restless leg syndrome, muscle cramps, purpuric petechiae, spider-web phlebomegaly, heaviness of legs, paresthesiae and lower limb nerve pain, pooling of blood in inferior limbs and reduced venous return, venous stasis, orthostatic intolerance, thrombophlebitis and thrombus formation in veins and embolism thereof resulting in cardio pulmonary vascular events. The principle extends similarly through microcirculatory dysfunction in other organs also such as lung, heart, liver, brain, kidney retina, pancreas, testis, rectum, spinal cord, muscles, sympathetic and parasympathetic ganglia, and nerves. It also discloses administering vitamin K analogues for improving skin, hair and nail health in subjects in need thereof.

### BRIEF DESCRIPTION OF THE DISCLOSURE

The present disclosure is directed to a composition for use in treating or preventing delayed onset muscle soreness, including administering to a subject in need thereof a composition including vitamin K2 and at least one anticoagulant. The present disclosure is also directed to a combination for use in treating or preventing delayed onset muscle soreness, comprising administering to a subject in need thereof a combination comprising vitamin K2 and at least one anticoagulant.

### BRIEF DESCRIPTION OF THE DRAWINGS

Fig. 1 shows the results of the study described in Example I(a).
Fig. 2 shows the results of the study described in Example I(b).
Fig. 3 shows the results of the study described in Example I(c).
Fig. 4 shows the results of the study described in Example I(d).
Fig. 5 shows the results of the study described in Example I(e).
Fig. 6 shows the results of the study described in Example II.
Fig. 7 shows the results of the study described in Example III(a).
Fig. 8 shows the results of the study described in Example III(b).
Fig. 9 shows the results of the study described in Example IV(a).
Fig. 10 shows the results of the study described in Example IV(b).

### DETAILED DESCRIPTION OF THE DISCLOSURE

The present disclosure is directed to methods of treating a subject prone to and/or suffering from unacceptable blood clotting, reduced blood flow, oxidative stress, unacceptably low ATP production, combinations thereof, and/or symptoms thereof, the method comprising administering to a subject in need thereof a combination of at least one anticoagulant and vitamin K. According to some aspects, the subject may suffer from a condition that results from and/or is responsible for unacceptable blood clotting, oxidative stress, unacceptably low ATP production, and/or reduced blood flow. According to some aspects, the amount of the at least one anticoagulant and vitamin K is sufficient to prevent unacceptable blood clotting; reduce and/or eliminate the number and/or severity of blood clots in a subject's body; prevent, reduce, and/or eliminate oxidative stress; increase a subject's ATP production; prevent unacceptably low ATP production; increase a subject's blood flow; prevent reduced blood flow; or a combination thereof.

The present disclosure is directed to methods of preventing, reducing, and/or eliminating oxidative stress. As used herein, the phrase "oxidative stress" refers to an imbalance between a subject's systemic manifestation of reactive oxygen species and the subject's ability to detoxify the reactive intermediates and/or to repair the resulting damage. In should be understood that this imbalance increases a subject's risk of undesirable complications, including pain (e.g., muscle pain), soreness (e.g., muscle soreness), and combinations thereof. According to some aspects, oxidative stress may correspond with high levels of free radicals, particularly reactive oxygen species such as peroxides, in a subject's body.

According to some aspects, oxidative stress may result at least in part from a condition wherein energy production by muscle cells is compromised, including, but not limited to, cardiorespiratory diseases. According to some aspects, subjects who may benefit from the claimed invention include those suffering from a cardiorespiratory disease, examples of which include, but are not limited to, coronary heart diseases, strokes, transient ischaemic attacks, peripheral arterial diseases, and aortic diseases.

Further, oxidative stresses may inhibit ATP production, thereby resulting in unacceptably low ATP production. According to some aspects, the claimed invention of the present disclosure may increase ATP production in a subject in need thereof. According to some aspects, by preventing, reducing, and/or eliminating oxidative stress in a subject, unacceptably low ATP production in the subject may be prevented.

According to one aspect, the invention provides a composition for use in treating or preventing delayed onset muscle soreness, comprising administering to a subject in need thereof a composition comprising vitamin K2 and at least one anticoagulant.

According to a further aspect, the invention provides a combination for use in treating or preventing delayed onset muscle soreness, comprising administering to a subject in need thereof a combination comprising vitamin K2 and at least one anticoagulant.

Preferably, the subject suffers from oxidative stress, unacceptably low ATP production, unacceptably low blood flow, or a combination thereof.

A further aspect of the invention provides a formulation comprising a combination of vitamin K2 and at least one anticoagulant, wherein the at least one anticoagulant comprises a first anticoagulant that is configured to inhibit free Factor Xa and/or Factor Xa bound in a prothrombinase complex in a subject.

Preferably, the first anticoagulant is selected from the group consisting of rivaroxaban, apixaban, and dabigatran etexilate.

Preferably, the rivaroxaban is administered in an amount of between about 15 and 20 mg/day.

Preferably, the apixaban is administered in an amount of between about 2.5 and 5 mg/day.

Preferably, the dabigatran etexilate is administered in an amount of between about 150 and 300 mg/day.The combination according to the present disclosure comprises vitamin K2. Those skilled in the art will understand that vitamin K and derivatives thereof refer to one or more compounds of Formula 1 and their pharmaceutically or nutritionally acceptable salts: 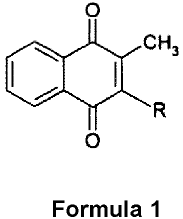 wherein R may be any covalently linked organic group including polyisoprenoid residues, esters, ethers, and thiol adducts. According to some aspects, the vitamin K2 comprised by the combination may be a compound having Formula 2: 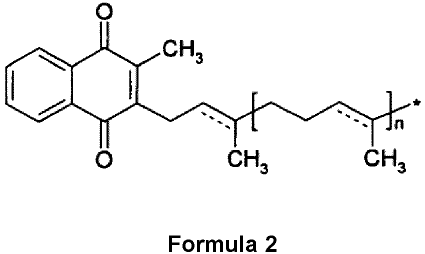 in which n is an integer from 1 to 12 and in which the broken lines indicate the optional presence of a double bond.

According to some aspects, the vitamin K2 comprised by the combination may be a menaquinone selected from the group consisting of short-chain menaquinones (e.g., MK-1, MK2, MK-3, and MK-4) and long-chain menaquinones (e.g., MK-5, MK-6, MK-7, MK-8, and MK-9), or a combination thereof. Those skilled in the art will understand that menaquinones are abbreviated MK-n, wherein M represents menaquinone, K represents vitamin K2, and n represents the number of isoprenoid side chain residues.

Sources of vitamin K2 which may be useful according to aspects of the present disclosure include, but are not limited to, different forms of vitamin K2 including synthetic MK-4, MK-5, MK-6, MK-7, MK-8,MK-9, MK-10, MK-11, MK12, and MK-13, natto (i.e., food prepared from fermented soybean), fermented foods, dairy products, and combinations thereof.

The combination according to the present disclosure further comprises at least one anticoagulant. According to some aspects, an "anticoagulant" refers to an agent that manipulates the blood coagulation process in a subject, and particular, provides an anti-clotting effect. According to some aspects of the present disclosure, the at least one anticoagulant comprises an anticoagulant that inhibits free Factor Xa and/or Factor Xa bound in the prothrombinase complex. Inhibition of Factor Xa may interrupt the intrinsic and extrinsic pathway of the blood coagulation cascade, inhibiting both thrombin formation and development of thrombi. Examples of anticoagulants useful according to the present disclosure include, but are not limited to, rivaroxaban, apixaban, and dabigatran etexilate.

According to some aspects, the combination may be free of an anticoagulant that is contradicted for use with vitamin K, also referred to herein as "vitamin K contradicted anticoagulants." Vitamin K contradicted anticoagulants include classes of anticoagulants that provide an unacceptable effect when administered with vitamin K. For example, some classes of anticoagulants function by inhibiting the vitamin K-dependent synthesis of biologically active forms of the calcium-dependent clotting factors II, VII, IX, and/or X and/or the regulatory factors protein C, protein S, and/or protein Z. As such, administration of vitamin K2 with such anticoagulants may reduce the desired manipulation of the blood coagulation process. Examples of vitamin K contradicted anticoagulants include, but are not limited to, warfarin, coumatetralyl, phenprocoumon, acenocoumarol, dicoumarol, tioclomarol, brodifacoum, and combinations thereof.

The use of the composition or combination according to the present disclosure may comprise administering a first amount of vitamin K2 in combination with a second amount of the at least one anticoagulant. It should be understood that a "combination" or "in combination" as used herein may refer to simultaneous and/or sequential administration. For example, the use may comprise administering the first amount of vitamin K2 followed by administering the second amount of the at least one anticoagulant before or during the time that the first amount of vitamin K2 is (or becomes) active in the body, or vice versa. According to some aspects, the use may comprise administering the first amount of vitamin K2 simultaneously or about simultaneously with the second amount of at least one anticoagulant.

The first and second amounts may be administered in one or more daily doses. Those skilled in the art will understand that a "dose" refers to the quantity of an agent administered at a particular point in time. According to some aspects, the first amount of vitamin K2 may be delivered in a single daily dose or may be delivered over the course of multiple doses per day. For example, the first amount of vitamin K2 may be administered in one, two, three, four, five, or more daily doses, wherein each dose contains the same or a different amount of vitamin K2 with respect to one or more other doses. Similarly, the second amount of at least one anticoagulant may be administered in one, two, three, four, five, or more daily doses, wherein each dose contains the same or a different amount of at least one anticoagulant with respect to one or more other doses. According to some aspects, each dose of vitamin K2 may independently be administered simultaneously with or sequentially with respect to a dose of at least one anticoagulant. According to some aspects, each dose of vitamin K2 may be administered simultaneously with or about simultaneously with a dose of the at least one anticoagulant.

The first amount of vitamin K2 may be a therapeutically effective amount. According to some aspects, the therapeutically effective amount of vitamin K2 may refer to an amount of vitamin K2 that, when administered in combination with the at least one anticoagulant, reduces and/or eliminates the number and/or severity of blood clots in a subject's body, reduces and/or eliminates oxidative stress, increases a subject's ATP production, increases a subject's blood flow, or a combination thereof.

The therapeutically effective amount of vitamin K2 may refer to an amount of vitamin K2 that enhances the anti-clotting ability of the at least one anticoagulant such that the at least one anticoagulant provides a greater anti-clotting effect than the anti-clotting effect observed when the at least one anticoagulant is administered without the vitamin K2. Alternatively or additionally, the therapeutically effective amount of vitamin K2 may be an amount of vitamin K2 that lowers the therapeutically effective amount of the at least one anticoagulant. In particular, the therapeutically effective amount of vitamin K2 may be an amount wherein a lesser amount of the at least one anticoagulant is required to prevent unacceptable blood clotting; reduce and/or eliminate the number and/or severity of blood clots in a subject's body; prevent, reduce, and/or eliminate oxidative stress; increase a subject's ATP production; prevent unacceptably low ATP production; increase a subject's blood flow; prevent reduced blood flow; or a combination thereof in a subject when compared with the amount of the at least one anticoagulant required to provide a comparable or the same effect in the subject when vitamin K2 is not administered. Alternatively or additionally, the therapeutically effective amount of vitamin K2 may refer to an amount of vitamin K2 sufficient to reduce and/or prevent hemorrhaging by activating blood-clotting factors and/or to provide acceptable carboxylation of two bone matrix proteins necessary for acceptable bone metabolism.

According to some aspects, the therapeutically effective amount of vitamin K2 may correspond to a dosage of between about 10 and 2000 µg/day, optionally between about 50 and 1000 µg/day, optionally between about 150 to 500 µg/day. According to some aspects, the therapeutically effective amount of vitamin K2 may correspond to a dosage of between about 10 and 16000 µg/week, optionally between about 70 and 14000 µg/week, optionally between about 350 to 7000 µg/week.

The second amount of the at least one anticoagulant may be a therapeutically effective amount. According to some aspects, the therapeutically effective amount of the at least one anticoagulant may refer to an amount of the at least one anticoagulant that, when administered in combination with vitamin K2, prevents unacceptable blood clotting; reduces and/or eliminate the number and/or severity of blood clots in a subject's body; prevents, reduces, and/or eliminates oxidative stress; increases a subject's ATP production; prevents unacceptably low ATP production; increases a subject's blood flow; prevents reduced blood flow; or a combination thereof. According to some aspects, the therapeutically effective amount of the at least one anticoagulant may be less than the amount of the at least one anticoagulant necessary to provide an acceptable anti-clotting effect when the at least one anticoagulant is administered without the vitamin K2.

According to some aspects, the therapeutically effective amount of the at least one anticoagulant may correspond to a dosage of between about 5 and 30 mg/day, optionally between about 15 and 20 mg/day. According to some aspects, the therapeutically effective amount of the at least one anticoagulant may correspond to a dosage of between about 1 and 10 mg/day, optionally between about 2.5 and 5 mg/day. According to some aspects, the therapeutically effective amount of the at least one anticoagulant may correspond to a dosage of between about 50 and 400 mg/day, optionally between about 150 and 300 mg/day. It should be understood that the therapeutically effective amounts of the at least one anticoagulant as described herein may refer to the total amount of anticoagulant in the combination or they may refer to an amount of one of the at least one anticoagulants in the combination.

It is believed that the combination therapy of the invention provides synergistic results, that is, the effects of the combination are greater than (or provide benefits that are different than) either therapy alone.

According to some aspects, the combination may be administered enterally, parenterally, topically, or a combination thereof. It should be understood that enteral administration includes oral, buccal, enteral, and intragastric administration. Parenteral administration includes any form of administration in which the combination is absorbed into the blood stream without involving absorption via the intestines. Examples of parenteral administration include, but are not limited to intramuscular, intravenous, intraperitoneal, intraocular, subcutaneous, and intraarticular administration, and combinations thereof.

The use comprises administering the combination to a subject in need thereof. According to some aspects, the subject may suffer from Afib and/or a cardiorespiratory disease. According to some aspects, the subject may suffer from unacceptable blood clotting, reduced blood flow, oxidative stress, unacceptably low ATP production, combinations thereof, and/or symptoms thereof. Subjects who may benefit from the treatment as described herein may additionally be those presenting with pulmonary embolism, joint replacement (e.g., hip or knee replacement), deep vein thrombosis, or a combination thereof. Other subjects who may benefit from the treatment as described herein may be subjects who suffer from and/or are prone to muscle soreness as a result of exercise, for example, eccentric exercise. For example, according to some aspects, the use may comprise administering the combination to a subject suffering from delayed onset muscular soreness. According to some aspects, the use may comprise administering the combination to a subject prior to exercise. The combination may be administered to the subject for a period of between 1 hour to 50 weeks prior to exercise, optionally between about 1 day to 40 days prior to exercise, optionally between about 1 day to 7 days prior to exercise. The subject may be a mammal such as a human, pet animal (e.g., dogs, cats), laboratory animal (e.g., rats, mice), or a farm animal (e.g., sheep, horses, cows).

The present disclosure is also directed to a composition comprising the combination as described herein. The composition may be in the form of a tablet (coated or uncoated), capsule (hard or soft), spray, soft gel, gummy, dragee, lozenge, oral solution, suspension, dispersion, syrup, sterile parenteral preparation, and/or a combination thereof. It should be understood that the composition may comprise one or more forms as described herein, wherein each form includes one or both components (i.e., the vitamin K and the at least one anticoagulant) of the combination.

The composition may additionally include pharmaceutically acceptable additives, carriers, excipients, or a combination thereof. Examples of excipients include, but are not limited to, diluents such as calcium carbonate, sodium carbonate, lactose, calcium phosphate, and sodium phosphate; granulating and disintegrating agents such as cornstarch or alginic acid; binding agents such as starch gelatin or acacia; effervescents; lubricating agents such as magnesium stearate, stearic acid, and talc; and combinations thereof. Examples of additives including, but are not limited to, preservatives, chelating agents, effervescing agents, natural and artificial sweeteners, flavoring agents, coloring agents, taste masking agents, acidulants, emulsifiers, thickening agents, suspending agents, dispersing agents, wetting agents, antioxidants, and combinations thereof.

The composition may be provided as a fortified food or beverage. Examples of fortified food and beverages include, but are not limited to, juice drinks, dairy drinks, powdered drinks, sports drinks, mineral water, soy beverages, hot chocolate, malt drinks, biscuits, bread, crackers, confectioneries, chocolate, chewing-gum, margarines, spreads, yogurts, breakfast cereals, snack bars, meal replacements, protein powders, desserts, medical nutrition tube feeds, nutritional supplements, and combinations thereof.

The present disclosure is also directed to a kit containing the compositions as described herein along with instructions for administering the combination as described herein.

This written description uses examples to disclose the invention, including the preferred embodiments, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims. Aspects from the various embodiments described, as well as other known equivalents for each such aspect, can be mixed and matched by one of ordinary skill in the art to construct additional embodiments and techniques in accordance with principles of this application.

While the aspects described herein have been described in conjunction with the example aspects outlined above, various alternatives, modifications, variations, improvements, and/or substantial equivalents, whether known or that are or may be presently unforeseen, may become apparent to those having at least ordinary skill in the art. Accordingly, the example aspects, as set forth above, are intended to be illustrative, not limiting. Various changes may be made without departing from the spirit and scope of the disclosure. Therefore, the disclosure is intended to embrace all known or later-developed alternatives, modifications, variations, improvements, and/or substantial equivalents.

Reference to an element in the singular is not intended to mean "one and only one" unless specifically so stated, but rather "one or more." All structural and functional equivalents to the elements of the various aspects described throughout this disclosure that are known or later come to be known to those of ordinary skill in the art are expressly incorporated herein. Moreover, nothing disclosed herein is intended to be dedicated to the public.

Further, the word "example" is used herein to mean "serving as an example, instance, or illustration." Any aspect described herein as "example" is not necessarily to be construed as preferred or advantageous over other aspects. Unless specifically stated otherwise, the term "some" refers to one or more. Combinations such as "at least one of A, B, or C," "at least one of A, B, and C," and "A, B, C, or any combination thereof" include any combination of A, B, and/or C, and may include multiples of A, multiples of B, or multiples of C. Specifically, combinations such as "at least one of A, B, or C," "at least one of A, B, and C," and "A, B, C, or any combination thereof" may be A only, B only, C only, A and B, A and C, B and C, or A and B and C, where any such combinations may contain one or more member or members of A, B, or C.

As used herein, the term "about" and "approximately" are defined to being close to as understood by one of ordinary skill in the art. In one non-limiting embodiment, the term "about" and "approximately" are defined to be within 10%, preferably within 5%, more preferably within 1%, and most preferably within 0.5%.

### EXAMPLES

### Example I(a): Vitamin K Uptake in Vascular Smooth Muscle Cells (VSMCs)

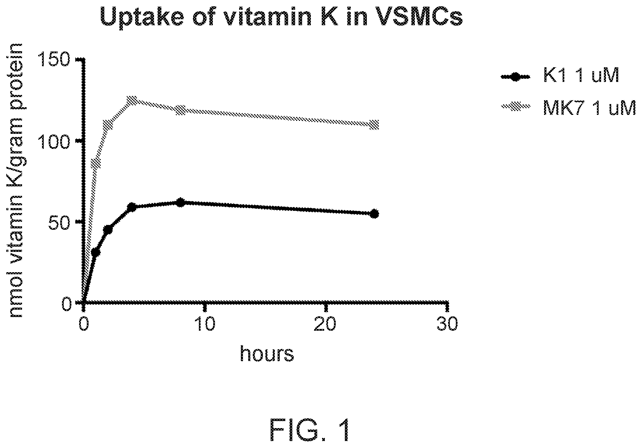

Vascular smooth muscle cells (VSMCs) were grown until 80% confluence, and then the medium was changed to medium supplemented with 1 µM vitamin K2 (MK-7) or 1 µM vitamin K1. Cells were grown for 24 hours, and cells were harvested at time points 0, 1, 2, 4, 8, and 24 hours. Cells were lysed and vitamin K content was measured by HPLC. As shown in Fig. 1, the vitamin K uptake observed in VSMCs exposed to 1 µM vitamin K2 (MK-7) was superior to the vitamin K uptake observed in VSMCs exposed to 1 µM vitamin K1.

### Example I(b): Oxidative Stress in VSMCs

It is known that the level of reactive oxygen species in cells can be measured by measuring the conversion of 2',7'-dichlorofluorescein diacetate (DCFDA) to the fluorescent dye 2',7'-dichlorofluorescein (DCF). The fluorescence generated by cells subjected to DCFDA is directly proportional to the amount of oxidized DCFDA to DCF.

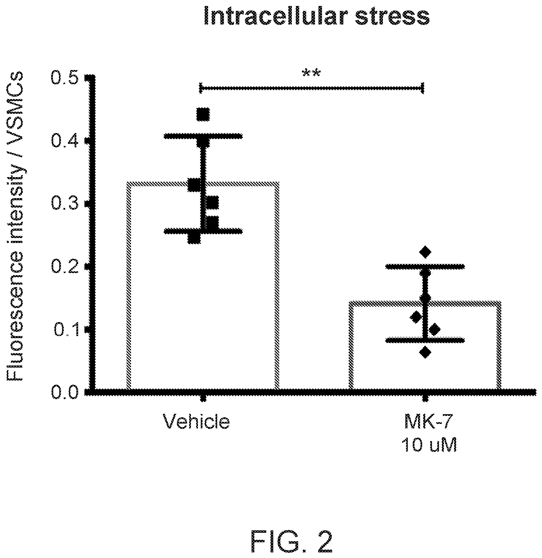

To perform the study, VSMCs (10,000 cells/well) were plated in a 96-well plate and left to adhere overnight. Next, cells were incubated for 1 hour with 20 µM DCFDA. The medium was then changed to medium supplemented with 10 µM vitamin K2 (MK-7). To visualize intracellular oxidative stress, fluorescence was measured for 6 hours and fluorescence intensity was normalized for cell count. As shown in Fig. 2, vitamin K2 (MK-7) clearly reduced the oxidative stress in VSMCs.

### Example I(c): Warfarin-Induced Oxidative Stress in VSMCs

This study was performed to determine if vitamin K2 (MK-7) reduces warfarin-induced oxidative stress. To perform the study, VSMCs (10,000 cells/well) were plated in a 96-well plate and left to adhere overnight. Next, cells were incubated for 24 hours with 100 µM warfarin. The next day, 20 µM DCFDA was added to the cells for 1 hour. The cells were then incubated for 5 hours with normal medium (baseline), vitamin K2 (MK-7), or warfarin. Fluorescence was measured and fluorescence intensity was normalized for cell count.

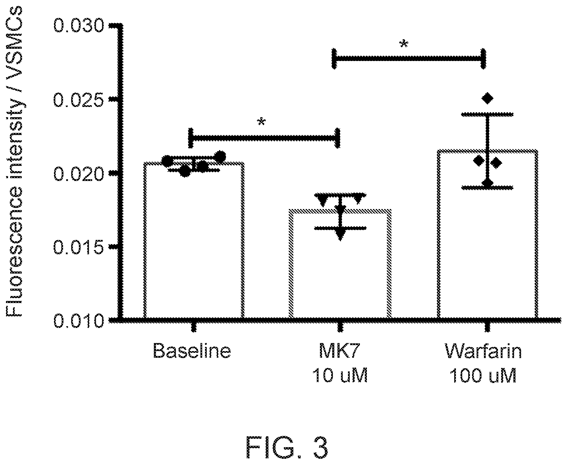

As shown in Fig. 3, vitamin K2 (MK-7) reduced not only normal oxidative stress in VSMCs but also counteracted the warfarin-induced oxidative stress.

### Example I(d): Warfarin-Induced Oxidative Stress in VSMCs

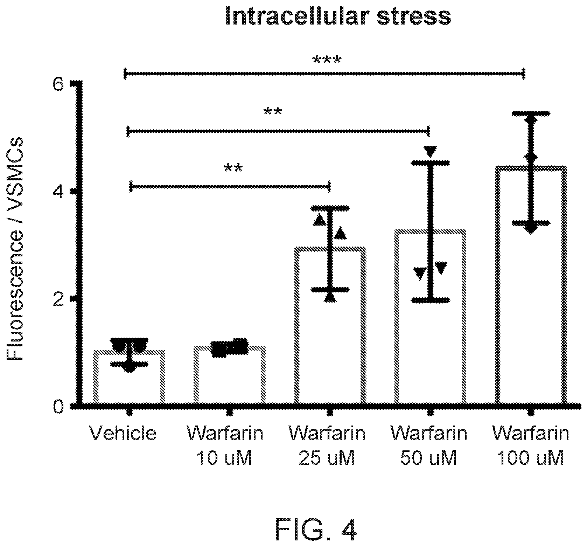

In this study, vitamin K metabolism was blocked using the vitamin K-antagonist warfarin. The study was conducted to investigate whether interference with vitamin K metabolism would cause intracellular oxidative stress. To perform the study, VSMCs (10,000 cells/well) were plated in 96-well plates and left to adhere overnight. Next, cells were incubated for 1 hour with 20 µM DCFDA. The medium was then changed to a medium supplemented with different concentrations of warfarin ranging from 10 to 100 µM, and fluorescence intensity was measured over 6 hours. Fluorescence was normalized for number of cells. Fig. 4 shows the results of this study, specifically that warfarin did cause intracellular oxidative stress.

### Example I(e): Hypoxia Induced Oxidative Stress

This study was performed to determine whether intracellular oxidative stress in VSCMs is derived from mitochondrial dysfunction. To perform this study, VSMCs were incubated with cobalt chloride, a known stabilizer of HIF1a. Cobalt chloride binding to HIF1a prevents the degradation of HIF1a and thus results in a cellular hypoxia state. HIF1a has been implicated in cancer biology as well as a number of other pathophysiologies, specifically in areas of vascularization and angiogenesis, energy metabolism, cell survival, and tumor invasion. Under normal circumstances, after injury, HIF1a is degraded by the enzyme prolyl hydroxylase (PHD). The continued up-regulation of HIF1a (mimicked by cobalt chloride) promotes tumor growth and metastasis through its role in initiating angiogenesis and regulating cellular metabolism to overcome hypoxia. Hypoxia promotes apoptosis in both normal and tumor cells. Additionally, hypoxia generates significant intracellular oxidative stress.

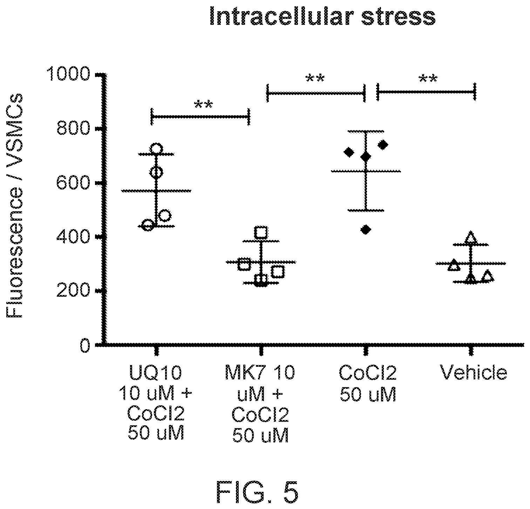

To perform the study, VSMCs (10,000 cells/well) were plated in a 96-well plate and left to adhere overnight. Next, the medium was changed to a medium supplemented with 50 µM CoCl₂ for 19 hours. The next day, cells were washed and incubated for 1 hour with 20 µM DCFDA in the presence of a vehicle (solvent for CoCl₂ and vitamin K2 (MK-7)/UQ10), 10 µM vitamin K2 (MK-7), or 10 µM UQ10 in medium. The medium was then changed to a medium supplemented with vehicle, vitamin K2 (MK-7)/UQ10, or CoCl₂, and fluorescent was measured for 6 hours. Fluorescence intensity was normalized for cell count. Fig. 5 shows the results of this study.

### Example I(f): Conclusions

Based on the studies described in Examples I(a)-I(e), it was concluded that VSMCs are able to take up vitamin K2 (MK-7) very efficiently and significantly better than vitamin K1. Interference with vitamin K metabolism (i.e., using warfarin to block the redox recycling of vitamin K) results in increased intracellular oxidative stress. The addition of vitamin K2 (MK-7) counteracts intracellular oxidative stress, both under normal conditions as well as under warfarin-induced oxidative stress conditions. Additionally, the HIF1a stabilizing cobalt chloride (inducing chronic hypoxia, as in during endurance sporting) induces increased oxidative stress, which might be detrimental for the muscle tissue. Also, under these circumstances, vitamin K2 (MK-7) seems to counteract hypoxia induced oxidative stress, indicative for improved mitochondrial activity. This effect was not found for UQ10, a known intermediate in the mitochondrial respiratory chain.

### Example II: Vitamin K2 (MK-7) and ATP Pathway

This study was performed in order to determine the effect of vitamin K2 (MK-7) on warfarin-induced oxidative stress in muscles. To perform this study, human vascular smooth muscle cells (hVSMCs; 5,000 cells/well) were plated in 96-well dark plates and left to adhere overnight. Next, cells were incubated for 24 hours with either vehicle only (as blank control), warfarin (10 or 50 µM), vitamin K2 (MK-7) (10 µM), or a combination of warfarin (50 µM) and vitamin K2 (MK-7) (10 µM). Hoechst was added to correct for cell number (1 µg/mL). Next, ATP was measured using mammalian lysis buffer for 5 minutes after which 50 µL ATP substrate solution was added to the wells and incubated for 10 minutes in a dark environment. Finally, medium was transferred to a white plate to measure luminescence.

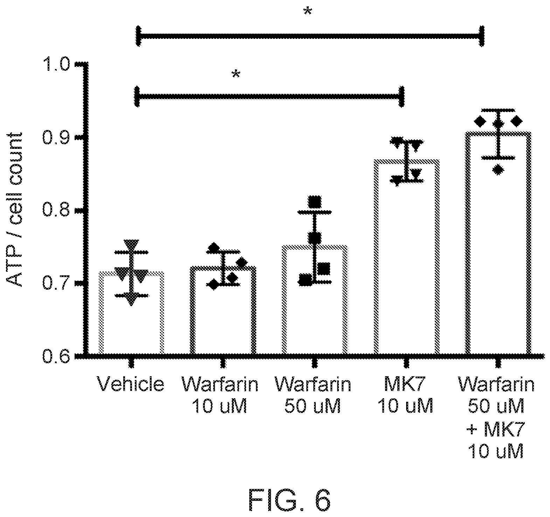

Fig. 6 shows the results of this study. Based on these results, it was determined that vitamin K2 (MK-7) counteracted the oxidative stress induced by warfarin and increased cell ATP. It was hypothesized that this stress was derived from the blockage of the VKOR (Vitamin K-Epoxide Reductase) enzyme which lies in the endoplasmic reticulum (ER) compartment. Clearly, warfarin does not affect ATP generation in VSMCs. This suggests that the increased intracellular stress caused by warfarin is not an effect on mitochondrial stress, but rather via the VKOR enzyme which is located in the ER. Here, the effect of vitamin K2 (MK-7) is well described as posttranslational carboxylation of vitamin K dependent proteins, which takes place in the ER, where vitamin K is recycled via the VKOR enzyme.

### Example III(a): ATP Increase

This study was performed to determine if vitamin K2 (MK-7) affects ATP generation. ATP is generated via glycolysis in the cytoplasm and via both the Krebs-cycle and oxidative phosphorylation in the mitochondria. ATP generation via glycolysis delivers only 2 ATP, whereas ATP generation in the mitochondria generates some 32 ATP.

To perform this study, VSCMs (5000 cells/well) were plated in 96-well plates and left to adhere overnight. Next, cells were incubated for 24 hours with either vehicle, vitamin K2 (MK-7) (10 µM), or UQ10 (10 µM). Hoechst was added to correct for cell number (1 µg/mL). Next, ATP was measured using mammalian lysis buffer for 5 minutes after which 50 µL ATP substrate solution was added to the wells and incubated for 10 minutes in a dark environment. Finally, the medium was transferred to a white plate to measure luminescence

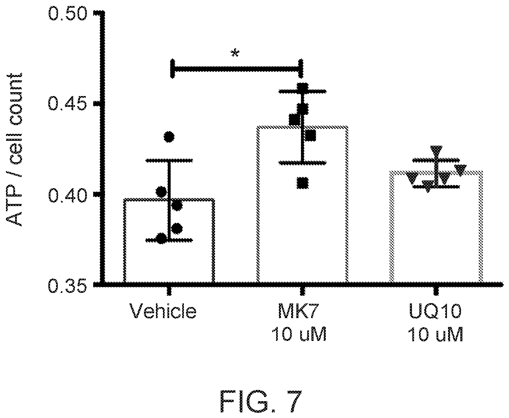

Fig. 7 shows the results of this study. Based on these results, it was determined that compared to the vehicle (solvent for vitamin K2 (MK-7) or UQ10), only vitamin K2 (MK-7) increased ATP production in VSMCs significantly (p=0.014). This can either be due to ATP synthesis via glycolysis or via oxidative phosphorylation in the mitochondria.

### Example III(b): ATP Increase

This study was performed to further investigate the origin of ATP production that is influenced by vitamin K2 (MK-7). To perform this study, VSCMs (5000 cells/well) were plated in 96-well plates and left to adhere overnight. Next, cells were incubated for 48 hours with either vehicle, CoCl₂ (100 µM), vitamin K2 (MK-7) (10 µM), or UQ10 (10 µM). Hoechst was added to correct for cell number (1 µg/mL). Next, ATP was measured using mammalian lysis buffer for 5 minutes, after which 50 µL ATP substrate solution was added to the wells and incubated for 10 minutes in a dark environment. Finally, medium was transferred to a white plate to measure luminescence.

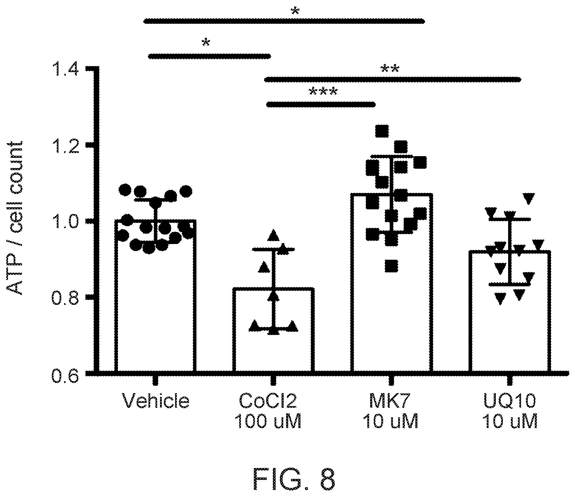

Fig. 8 shows the results of this study. Based on these results, it was determined that CoCl₂ (via stabilizing HIF1a) decreased ATP production. The hypoxia that is induced by CoCl₂ decreased ATP production via oxidative phosphorylation, as this pathway is oxygen dependent. Again, vitamin K2 (MK-7) increased ATP production slightly, though significant compared to vehicle. Compared to CoCl₂, both vitamin K2 (MK-7) and UQ10 displayed significant higher ATP levels, indicative for an effect via mitochondria.

### Example IV(a): Vitamin K2 (MK-7) and Hypoxia

This study was performed to investigate whether vitamin K2 (MK-7) can prevent the hypoxia-induced decrease in ATP (CoCl₂ treatment, thereby stabilization of HIF1a). To perform this study, VSMCs were co-treated with with CoCl₂ and vitamin K2 (MK-7).

First, VSCMs (5000 cells/well) were plated in 96-well plates and left to adhere overnight. Next, cells were incubated for 24 hours with either CoCl₂ (100 µM) or CoCl₂ and vitamin K2 (MK-7) or UQ10 together (100 and 10 µM, respectively). Next, ATP was measured and corrected for cell numbers. CoCl₂ values were set as relative to CoCl₂ co-treated with vitamin K2 (MK-7) or UQ10. Hoechst was added to correct for cell number (1 µg/mL). Next, ATP was measured using mammalian lysis buffer for 5 minutes after which 50 µL ATP substrate solution was added to the wells and incubated for 10 minutes in a dark environment. Finally, medium was transferred to a white plate to measure luminescence.

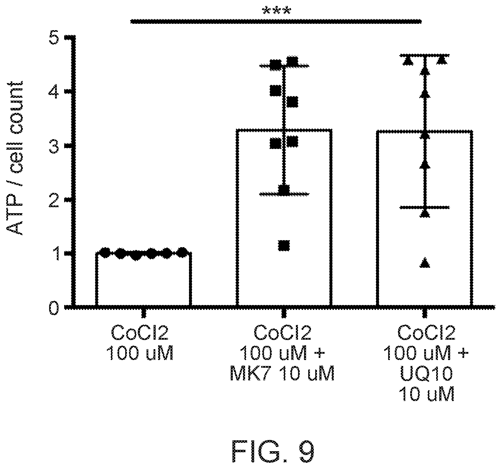

Fig. 9 shows the results of this study. Based on the data shown in Fig. 9, it was concluded that the co-treatment of VSMCs with CoCl₂ could (partially) prevent the decreased ATP generation, which indicates that vitamin K2 (MK-7) directly impacts mitochondrial function and ATP generation. It was also concluded that vitamin K2 (MK-7) reduces the impact of accumulated calcium in cells associated with DOMS.

### Example IV(b): Vitamin K2 (MK-7) and Hypoxia

This study was performed to investigate the effect of vitamin K2 (MK-7) on ATP over time. It is known that all cells, including VSMCs, proliferate. The term cell growth is used in the contexts of biological cell development and cell division (reproduction). When used in the context of cell division, it refers to growth of cell populations, where a cell, known as the "mother cell," grows and divides to produce two "daughter cells" (M phase). When used in the context of cell development, the term refers to increase in cytoplasmic and organelle volume (G1 phase), as well as increase in genetic material (G2 phase) following the replication during S phase. When a cell is quiescent, this phase is called G0. Cell populations go through a particular type of exponential growth called doubling. Each generation of cells should be twice as numerous as the previous generation. However, the number of generations only gives a maximum figure as not all cells survive in each generation.

To perform this study, first, VSCMs (5000 cells/well) were plated in 96-well plates and left to adhere overnight. Next, cells were incubated for indicated time points with vitamin K2 (MK-7) (10 µM). Hoechst was added to correct for cell number (1 µg/mL). Next, ATP was measured using mammalian lysis buffer for 5 minutes, after which 50 µL ATP substrate solution was added to the wells and incubated for 10 minutes in a dark environment. Finally, medium was transferred to a white plate to measure luminescence.

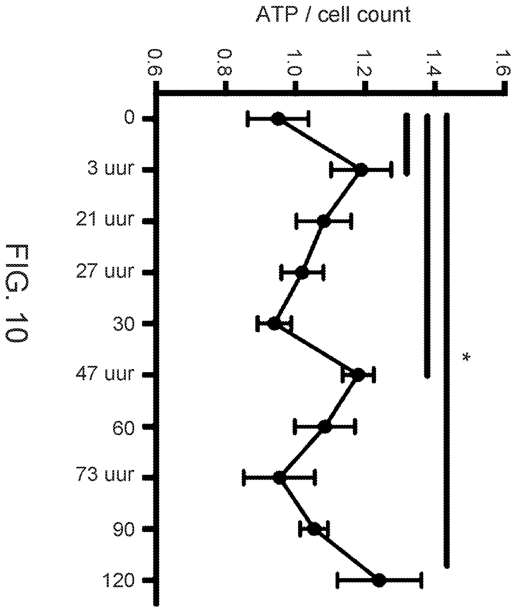

Fig. 10 shows the results of this study. As shown in Fig. 10, vitamin K2 (MK-7) stimulated ATP production in VSMCs, but vitamin K2 (MK-7) was depleted by cell growth. If additional vitamin K2 (MK-7) was added, ATP production was again stimulated. This indicates that a reservoir of vitamin K2 (MK-7) is necessary for cells to achieve maximal ATP production.

### Example V: Effects of Vitamin K2 (MK-7) on Anticoagulant Activity of the New Oral Anticoagulants (NOAC)

This experiment was conducted in order to evaluate the effects of vitamin K2 (MK-7) on the anticoagulant activity of the new oral anticoagulants (NOAC) rivaroxaban and dabigatran using the T-TAS Total Thrombus-formation Analysis System. The T-TAS Total Thrombus-formation Analysis System is a microchip-based flow chamber system capable of evaluating whole-blood thrombogenicity. It is a useful tool in monitoring the efficacy of anticoagulants on thrombus formation and predicting the risk of bleeding complications.

To perform the study, 45 wild-type male Sprague-Dawley and 45 transgenic male Ren-2 rats [TGR(mREN-2)27] at the age of 10-11 weeks at the beginning of the treatment period were purchased from animal facility IKEM-CEM Institute of Clinical and Experimental Medicine (Prague, Czech Republic). All animals in the experiment were housed in individually ventilated cages (2-3 rats per cage) and kept in the animal rooms of JCET at a relative humidity of 55±10%, a temperature of 22±2 °C, and a light cycle of 7 AM to 7 PM. The rats had free access to food and water. After delivery, animals were randomly divided into 10 experimental groups (5 or 10 rats per group), as indicated below in Table 1.

**Table 1: Experimental Groups**

| **Experimental Group** | **Rat Type** | **Treatment** | **Rats per Group** |
|---|---|---|---|
| 1 | Transgenic | **Control** | 5 |
| 2 | Transgenic | **Dabigatran** (7.5 mg/kg/day) | 10 |
| 3 | Transgenic | **Dabigatran** (7.5 mg/kg/day) + **vitamin K2 (MK-7)** (1.5 mg/kg/day) | 10 |
| 4 | Transgenic | **Rivaroxaban** (5.0 mg/kg/day) | 10 |
| 5 | Transgenic | **Rivaroxaban** (5.0 mg/kg/day) + **vitamin K2 (MK-7)** (1.5 mg/kg/day) | 10 |
| 6 | Wild-type | **Control** | 5 |
| 7 | Wild-type | **Dabigatran** (7.5 mg/kg/day) | 10 |
| 8 | Wild-type | **Dabigatran** (7.5 mg/kg/day) + **vitamin K2 (MK-7)** (1.5 mg/kg/day) | 10 |
| 9 | Wild-type | **Rivaroxaban** (5.0 mg/kg/day) | 10 |
| 10 | Wild-type | **Rivaroxaban** (5.0 mg/kg/day) + | 10 |
| | | **vitamin K2 (MK-7)** (1.5 mg/kg/day) | |

Four days before the start of the treatment, the first part of animals' body weight and food intake was measured in selected representative three cages (rats n=9) to calculate the appropriate amount of tested compounds required for addition to the diet to obtain the dosing indicated in Table 1. It was estimated that food intake was approximately 30 g of chow per rat per day. The animals were fed for two weeks with AIN-93G diet supplemented with rivaroxaban or dabigatran with/without Vitamin K2 (MK-7), as indicated in Table 1. The animals were then anesthetized in order to draw blood samples for further analysis. The body mass of the rats was measured at the beginning and at the end of treatment.

At the beginning and at the end of treatment, the food intake per day was measured in representative group of animals to monitor the daily dosing of tested compounds (per kg of body mass). The thrombus formation *in vitro* was measured in whole blood using a microchip-based flow chamber system according to manufacturers' protocol (Total Thrombus-formation Analyser System, T-TAS; Fujimori Kogyo Co., Ltd., Tokyo, Japan). For the analysis, citrated whole blood (480 µl) was mixed with 20 µl of 300 mM CaCl₂ solution containing 1.25 mg/ml CTI (Corn Trypsin Inhibitor). After mixing, blood samples were immediately perfused over a microchip coated with collagen and tissue thromboplastin at flow rates of 4 µl/min, which created initial wall shear rates of normal small veins (240 s⁻¹). Thrombus formation and breakdown within the microcapillaries caused flow disturbances that resulted in pressure increases and decreases, respectively.

The obtained flow pressure pattern for each sample was used to analyze thrombus formation based on the following estimated parameters:
Time to 10 kPa (T₁₀; min), which is the time required to reach 10 kPa from the baseline pressure and reflects the onset of thrombi formation;
Occlusion time (OT; min), which is the time required to reach 80 kPa from the baseline pressure and reflects nearly complete capillary occlusion; and
Area under the flow pressure curve for 30 min (under 80 kPa) after the start of the assay (AUC30), which reflects total thrombogenicity of whole blood.

Prothrombin Time (PT), activated Partial Thromboplastin Time (aPTT), Thrombin Time (TT) and fibrinogen levels (Clauss method) were determined according to the manufacturer's instructions using Coag-Chrom 3003 apparatus (Bio-ksel, Grudziadz, Poland). A more sensitive assay for fibrin generation was used on the basis of a method described previously (Bjornsson TD et al. Aspirin acetylates fibrinogen and enhances fibrinolysis. Fibrinolytic effect is independent of changes in plasminogen activator levels. J Pharmacol Exp Ther. 1989;250:154-161; He S et al. A simple and rapid laboratory method for determination of haemostasis potential in plasma. II. Modifications for use in routine laboratories and research work. Thromb Res. 2001;103:355-361.) and modified by Buczko et al. (Buczko W et al. Aspirin and the fibrinolytic response. Thromb Res. 2003;110:331-334.). Fibrin generation curves were created by recalcination of rat plasma samples directly in microplate wells with CaCl₂ (36 mmol/L) dissolved in Tris buffer (66-mmol/L Tris; 130-mmol/L NaCl; pH=7.4) at 37°C. Optical density increase in the wells (as a result of fibrin generation) was measured using a microplate reader (Biotek EL808, BioTek Instruments Inc., USA) in 1-minute intervals for 14 minutes and expressed as an area under the curve

As shown in Tables 2, dabigatran and rivaroxaban inhibited thrombus formation in the Sprague-Dawley rats, and the addition of vitamin K2 (MK-7) slightly increased the occlusion Time (OT) and thrombin time for both dabigatran and rivaroxaban by 5-7%, but did not prevent the formation of a clot (AUC). As shown in Table 3, dabigatran and rivaroxaban also inhibited thrombus formation in the TGR rats, and vitamin K2 (MK-7) did not modify this effect significantly. Based on this data, it was concluded that the addition of vitamin K may slow, but did not prevent, clot formation, thereby enhancing the activity of the anti-coagulants.

**Table 2: T-TAS Analysis Parameters Reflecting Thrombus Formation in Sprague-Dawley Rats**

| **Group** | **Treatment** | **T₁₀ (time, min)** | **OT (time, min)** | **AUC** |
|---|---|---|---|---|
| 6 | Control | 2.958 ± 0.29 | 5.062 ± 0.565 | 2088 ± 34.97 |
| 7 | Dabigatran | 3.386 ± 0.237 | 5.753 ± 0.587 | 2039 ± 32.5 |
| 8 | Dabigatran + Vitamin K2 (MK-7) | 3.607 ± 0.279 | 6.027 ± 0.317 | 2020 ± 23.5 |
| 9 | Rivaroxaban | 3.732 ± 0.266 | 6.133 ± 0.518 | 2011 ± 28.7 |
| 10 | Rivaroxaban+ | 3.912 ± 0.484 | 6.583 ± 0.807 | 1987 ± 149.9 |
| | Vitamin K2 MK-7 | | | |

**Table 3: T-TAS Analysis Parameters Reflecting Thrombus Formation in TGR Rats**

| **Group** | **Treatment** | **T₁₀ (time, min)** | **OT (time, min)** | **AUC** |
|---|---|---|---|---|
| 1 | Control | 3.824 ± 0.3617 | 6.514 ± 0.8366 | 2000 ± 40.62 |
| 2 | Dabigatran | 4.780 ± 0.7073 | 8.011 ± 1.317 | 1898 ± 80.66 |
| 3 | Dabigatran + Vitamin K2 (MK-7) | 4.720 ± 0.5242 | 8.016 ± 1.068 | 1906 ± 58.24 |
| 4 | Rivaroxaban | 4.996 ± 0.8216 | 8.324 ± 1.578 | 1876 ± 91.28 |
| 5 | Rivaroxaban+ Vitamin K2 MK-7 | 5.019 ± 0.6657 | 8.423 ± 1.385 | 1875 ± 75.94 |

## Claims

1. A composition for use in treating or preventing delayed onset muscle soreness, comprising administering to a subject in need thereof a composition comprising vitamin K2 and at least one anticoagulant.

2. A combination for use in treating or preventing delayed onset muscle soreness, comprising administering to a subject in need thereof a combination comprising vitamin K2 and at least one anticoagulant.

3. The composition according to claim 1 or the combination according to claim 2 for use in treating or preventing delayed onset muscle soreness, wherein the vitamin K2 is administered in an amount of between about 10 and 2000 µg/day, preferably between about 50 and 1000 µg/day, or between about 150 and 500 µg/day.

4. The composition according to any one of claims 1 and 3 or the combination according to any one of claims 2 to 3 for use in treating or preventing delayed onset muscle soreness, wherein the vitamin K2 is selected from the group consisting of MK-1, MK2, MK-3, MK-4, MK-5, MK-6, MK-7, MK-8, and MK-9, or a combination thereof.

5. The composition according to any one of claims 1, 3 and 4 or the combination according to any one of claims 2 to 4 for use in treating or preventing delayed onset muscle soreness, wherein the at least one anticoagulant comprises a first anticoagulant that inhibits free Factor Xa and/or Factor Xa bound in the prothrombinase complex.

6. The composition according to claim 5 or combination according to claim 5 for use in treating or preventing delayed onset muscle soreness, wherein the first anticoagulants is selected from the group consisting of rivaroxaban, apixaban, and dabigatran etexilate.

7. The composition according to claim 6 or combination according to claim 6 for use in treating or preventing delayed onset muscle soreness, wherein the first anticoagulants is rivaroxaban, preferably the rivaroxaban is administered in an amount of between about 15 and 20 mg/day.

8. The composition according to claim 6 or combination according to claim 6 for use in treating or preventing delayed onset muscle soreness, wherein the first anticoagulants is apixaban, preferably the apixaban is administered in an amount of between about 2.5 and 5 mg/day.

9. The composition according to claim 6 or combination according to claim 6 for use in treating or preventing delayed onset muscle soreness, wherein the first anticoagulants is dabigatran etexilate, preferably the dabigatran etexilate is administered in an amount of between about 150 and 300 mg/day.

10. The composition according to any one of claims 1 and 3 to 9 or the combination according to any one of claims 2 to 9 for use in treating or preventing delayed onset muscle soreness, wherein it is free of vitamin K contradicted anticoagulants, preferably free of vitamin K contradicted anticoagulants selected from the group consisting of warfarin, coumatetralyl, phenprocoumon, acenocoumarol, dicoumarol, tioclomarol, brodifacoum, and combinations thereof.

11. The composition according to any one of claims 1 and 3 to 10 for use in treating or preventing delayed onset muscle soreness, wherein the composition is in the form of a tablet (coated or uncoated), capsule (hard or soft), spray, soft gel, gummy, dragee, lozenge, oral solution, suspension, dispersion, syrup, sterile parenteral preparation, and/or a combination thereof.

12. The composition according to any one of claims 1 and 3 to 11 for use in treating or preventing delayed onset muscle soreness, wherein the composition additionally includes pharmaceutically acceptable additives, carriers, excipients, or a combination thereof.

13. The composition according to any one of claims 1 and 3 to 12 for use in treating or preventing delayed onset muscle soreness, wherein the composition is provided as a fortified food or beverage selected from the group consisting of juice drinks, dairy drinks, powdered drinks, sports drinks, mineral water, soy beverages, hot chocolate, malt drinks, biscuits, bread, crackers, confectioneries, chocolate, chewing-gum, margarines, spreads, yogurts, breakfast cereals, snack bars, meal replacements, protein powders, desserts, medical nutrition tube feeds, nutritional supplements, and combinations thereof.

14. The combination according to any one of claims 2 to 10 for use in treating or preventing delayed onset muscle soreness, wherein the vitamin K2 and the at least one anticoagulant are administered simultaneously or sequentially.

15. The combination according to any of claims 2 to 10 and 14 for use in treating or preventing delayed onset muscle soreness, wherein a first amount of vitamin K2 is delivered in a single daily dose or delivered over the course of multiple doses per day, wherein each dose contains the same or a different amount of vitamin K2, and wherein a second amount of the at least one anticoagulant is administered in one or more daily doses, wherein each dose contains the same or a different amount of the at least one anticoagulant.

## Patentansprüche

1. Zusammensetzung zur Verwendung beim Behandeln oder Vorbeugen von verzögert auftretendem Muskelkater, umfassend ein Verabreichen einer Zusammensetzung, umfassend Vitamin K2 und mindestens ein Antikoagulans, an ein Subjekt, das diese benötigt.

2. Kombination zur Verwendung beim Behandeln oder Vorbeugen von verzögert auftretendem Muskelkater, umfassend ein Verabreichen einer Kombination, umfassend Vitamin K2 und mindestens ein Antikoagulans, an ein Subjekt, das diese benötigt.

3. Zusammensetzung nach Anspruch 1 oder Kombination nach Anspruch 2 zur Verwendung beim Behandeln oder Vorbeugen von verzögert auftretendem Muskelkater, wobei das Vitamin K2 in einer Menge von zwischen etwa 10 und 2000 µg/Tag, vorzugsweise zwischen etwa 50 und 1000 µg/Tag oder zwischen etwa 150 und 500 µg/Tag verabreicht wird.

4. Zusammensetzung nach einem der Ansprüche 1 und 3 oder Kombination nach einem der Ansprüche 2 bis 3 zur Verwendung beim Behandeln oder Vorbeugen von verzögert auftretendem Muskelkater, wobei das Vitamin K2 ausgewählt ist aus der Gruppe, bestehend aus MK-1, MK2, MK-3, MK-4, MK-5, MK-6, MK-7, MK-8 und MK-9 oder einer Kombination davon.

5. Zusammensetzung nach einem der Ansprüche 1, 3 und 4 oder Kombination nach einem der Ansprüche 2 bis 4 zur Verwendung beim Behandeln oder Vorbeugen von verzögert auftretendem Muskelkater, wobei das mindestens eine Antikoagulans ein erstes Antikoagulans umfasst, das freien Faktor Xa und/oder Faktor Xa hemmt, der in dem Prothrombinase-Komplex gebunden ist.

6. Zusammensetzung nach Anspruch 5 oder Kombination nach Anspruch 5 zur Verwendung beim Behandeln oder Vorbeugen von verzögert auftretendem Muskelkater, wobei das erste Antikoagulans ausgewählt ist aus der Gruppe, bestehend aus Rivaroxaban, Apixaban und Dabigatranetexilat.

7. Zusammensetzung nach Anspruch 6 oder Kombination nach Anspruch 6 zur Verwendung beim Behandeln oder Vorbeugen von verzögert auftretendem Muskelkater, wobei das erste Antikoagulans Rivaroxaban ist, vorzugsweise das Rivaroxaban in einer Menge von zwischen etwa 15 und 20 mg/Tag verabreicht wird.

8. Zusammensetzung nach Anspruch 6 oder Kombination nach Anspruch 6 zur Verwendung beim Behandeln oder Vorbeugen von verzögert auftretendem Muskelkater, wobei das erste Antikoagulans Apixaban ist, vorzugsweise das Apixaban in einer Menge von zwischen etwa 2,5 und 5 mg/Tag verabreicht wird.

9. Zusammensetzung nach Anspruch 6 oder Kombination nach Anspruch 6 zur Verwendung beim Behandeln oder Vorbeugen von verzögert auftretendem Muskelkater, wobei das erste Antikoagulans Dabigatranetexilat ist, vorzugsweise das Dabigatranetexilat in einer Menge von zwischen etwa 150 und 300 mg/Tag verabreicht wird.

10. Zusammensetzung nach einem der Ansprüche 1 und 3 bis 9 oder Kombination nach einem der Ansprüche 2 bis 9 zur Verwendung beim Behandeln oder Vorbeugen von verzögert auftretendem Muskelkater, wobei sie frei von Vitamin K entgegenwirkenden Antikoagulanzien ist, vorzugsweise frei von Vitamin K entgegenwirkenden Antikoagulanzien, die ausgewählt sind aus der Gruppe, bestehend aus Warfarin, Coumatetralyl, Phenprocoumon, Acenocoumarol, Dicoumarol, Tioclomarol, Brodifacoum und Kombinationen davon.

11. Zusammensetzung nach einem der Ansprüche 1 und 3 bis 10 zur Verwendung beim Behandeln oder Vorbeugen von verzögert auftretendem Muskelkater, wobei die Zusammensetzung in Form einer Tablette (beschichtet oder unbeschichtet), einer Kapsel (hart oder weich), eines Sprays, eines Weichgels, eines Gummis, eines Dragees, einer Pastille, einer oralen Lösung, einer Suspension, einer Dispersion, eines Sirups, einer sterilen parenteralen Zubereitung und/oder einer Kombination davon ist.

12. Zusammensetzung nach einem der Ansprüche 1 und 3 bis 11 zur Verwendung beim Behandeln oder Vorbeugen von verzögert auftretendem Muskelkater, wobei die Zusammensetzung zusätzlich pharmazeutisch annehmbare Zusatzstoffe, Trägerstoffe, Hilfsstoffe oder eine Kombination davon beinhaltet.

13. Zusammensetzung nach einem der Ansprüche 1 und 3 bis 12 zur Verwendung beim Behandeln oder Vorbeugen von verzögert auftretendem Muskelkater, wobei die Zusammensetzung als angereichertes Nahrungsmittel oder Getränk bereitgestellt ist, das ausgewählt ist aus der Gruppe, bestehend aus Saftgetränken, Milchgetränken, pulverförmigen Getränken, Sportgetränken, Mineralwasser, Sojagetränken, heißer Schokolade, Malzgetränken, Keksen, Brot, Crackern, Süßwaren, Schokolade, Kaugummi, Margarine, Aufstrichen, Joghurts, Frühstückszerealien, Snackbars, Mahlzeitenersatz, Proteinpulvern, Desserts, medizinischen Ernährungssonden, Nahrungsergänzungsmitteln und Kombinationen davon.

14. Kombination nach einem der Ansprüche 2 bis 10 zur Verwendung beim Behandeln oder Vorbeugen von verzögert auftretendem Muskelkater, wobei das Vitamin K2 und das mindestens eine Antikoagulans gleichzeitig oder nacheinander verabreicht werden.

15. Kombination nach einem der Ansprüche 2 bis 10 und 14 zur Verwendung beim Behandeln oder Vorbeugen von verzögert auftretenden Muskelkater, wobei eine erste Menge an Vitamin K2 in einer einzelnen Tagesdosis oder über den Verlauf mehrerer Dosen pro Tag verabreicht wird, wobei jede Dosis die gleiche oder eine unterschiedliche Menge an Vitamin K2 enthält, und wobei eine zweite Menge des mindestens einen Antikoagulans in einer oder mehreren Tagesdosen verabreicht wird, wobei jede Dosis die gleiche oder eine unterschiedliche Menge des mindestens einen Antikoagulans enthält.

## Revendications

1. Composition destinée à être utilisée dans le traitement ou la prévention des douleurs musculaires d'apparition retardée, comprenant l'administration à un sujet qui en a besoin d'une composition comprenant de la vitamine K2 et au moins un anticoagulant.

2. Combinaison destinée à être utilisée dans le traitement ou la prévention des douleurs musculaires d'apparition retardée, comprenant l'administration à un sujet qui en a besoin d'une combinaison comprenant de la vitamine K2 et au moins un anticoagulant.

3. Composition selon la revendication 1 ou combinaison selon la revendication 2 destinée à être utilisée dans le traitement ou la prévention des douleurs musculaires d'apparition retardée, dans laquelle la vitamine K2 est administrée en une quantité comprise entre environ 10 et 2000 µg/jour, de préférence entre environ 50 et 1000 µg/jour, ou entre environ 150 et 500 µg/jour.

4. Composition selon l'une quelconque des revendications 1 et 3 ou combinaison selon l'une quelconque des revendications 2 à 3 destinée à être utilisée dans le traitement ou la prévention des douleurs musculaires d'apparition retardée, dans laquelle la vitamine K2 est choisie dans le groupe constitué par MK-1, MK2, MK-3, MK-4, MK-5, MK-6, MK-7, MK-8 et MK-9, ou une combinaison de ceux-ci.

5. Composition selon l'une quelconque des revendications 1, 3 et 4 ou combinaison selon l'une quelconque des revendications 2 à 4 destinée à être utilisée dans le traitement ou la prévention des douleurs musculaires d'apparition retardée, dans laquelle l'au moins un anticoagulant comprend un premier anticoagulant qui inhibe le Facteur Xa libre et/ou le Facteur Xa lié dans le complexe prothrombinase.

6. Composition selon la revendication 5 ou combinaison selon la revendication 5, destinée à être utilisée dans le traitement ou la prévention des douleurs musculaires d'apparition retardée, dans laquelle le premier anticoagulant est choisi dans le groupe constitué par le rivaroxaban, l'apixaban et l'étexilate de dabigatran.

7. Composition selon la revendication 6 ou combinaison selon la revendication 6 destinée à être utilisée dans le traitement ou la prévention des douleurs musculaires d'apparition retardée, dans laquelle le premier anticoagulant est le rivaroxaban, de préférence le rivaroxaban est administré en une quantité comprise entre environ 15 et 20 mg/jour.

8. Composition selon la revendication 6 ou combinaison selon la revendication 6 destinée à être utilisée dans le traitement ou la prévention des douleurs musculaires d'apparition retardée, dans laquelle le premier anticoagulant est l'apixaban, de préférence l'apixaban est administré en une quantité comprise entre environ 2,5 et 5 mg/jour.

9. Composition selon la revendication 6 ou combinaison selon la revendication 6 destinée à être utilisée dans le traitement ou la prévention des douleurs musculaires d'apparition retardée, dans laquelle les premiers anticoagulants sont le dabigatran etexilate, de préférence le dabigatran etexilate est administré en une quantité comprise entre environ 150 et 300 mg/jour.

10. Composition selon l'une quelconque des revendications 1 et 3 à 9 ou combinaison selon l'une quelconque des revendications 2 à 9 destinée à être utilisée dans le traitement ou la prévention des douleurs musculaires d'apparition retardée, dans laquelle elle est exempte d'anticoagulants contredits par la vitamine K, de préférence exempte d'anticoagulants contredits par la vitamine K choisis dans le groupe constitué par la warfarine, le coumatétralyle, la phénprocoumone, l'acénocoumarol, le dicoumarol, le tioclomarol, le brodifacoum et leurs combinaisons.

11. Composition selon l'une quelconque des revendications 1 et 3 à 10 destinée à être utilisée dans le traitement ou la prévention des douleurs musculaires d'apparition retardée, dans laquelle la composition est sous forme de comprimé (enrobé ou non enrobé), de capsule (dure ou molle), de spray, de gel mou, de gomme, de dragée, de pastille, de solution orale, de suspension, de dispersion, de sirop, de préparation parentérale stérile, et/ou d'une combinaison de ceux-ci.

12. Composition selon l'une quelconque des revendications 1 et 3 à 11, destinée à être utilisée dans le traitement ou la prévention des douleurs musculaires d'apparition retardée, dans laquelle la composition comprend en outre des additifs, des supports, des excipients ou une combinaison de ceux-ci pharmaceutiquement acceptables.

13. Composition selon l'une quelconque des revendications 1 et 3 à 12, destinée à être utilisée dans le traitement ou la prévention des douleurs musculaires d'apparition retardée, dans laquelle la composition est fournie sous la forme d'un aliment ou d'une boisson enrichi choisi dans le groupe constitué par les boissons à base de jus, les boissons laitières, les boissons en poudre, les boissons pour sportifs, l'eau minérale, les boissons à base de soja, le chocolat chaud, les boissons à base de malt, les biscuits, le pain, les crackers, les confiseries, le chocolat, le chewing-gum, les margarines, les pâtes à tartiner, les yaourts, les céréales pour petit-déjeuner, les snacks, les substituts de repas, les poudres de protéines, les desserts, les aliments pour tubes de nutrition médicale, les suppléments nutritionnels et leurs combinaisons.

14. Combinaison selon l'une quelconque des revendications 2 à 10 destinée à être utilisée dans le traitement ou la prévention des douleurs musculaires d'apparition retardée, dans laquelle la vitamine K2 et l'au moins un anticoagulant sont administrés simultanément ou séquentiellement.

15. Combinaison selon l'une quelconque des revendications 2 à 10 et 14, destinée à être utilisée dans le traitement ou la prévention des douleurs musculaires d'apparition retardée, dans laquelle une première quantité de vitamine K2 est administrée en une seule dose quotidienne ou administrée au cours de plusieurs doses par jour, dans laquelle chaque dose contient la même quantité ou une quantité différente de vitamine K2, et dans laquelle une seconde quantité de l'au moins un anticoagulant est administrée en une ou plusieurs doses quotidiennes, dans laquelle chaque dose contient la même quantité ou une quantité différente de l'au moins un anticoagulant.
